# EUROPEAN PATENT APPLICATION

(11) **EP 4 793 958 A1**
(43) Date of publication of application: **19.08.2026**
(21) Application number: 24877511.6
(22) Date of filing: 08.10.2024
(51) Int. Cl.: G16H 10/60, G06F 21/62, G16H 20/10, G16H 40/20, G06Q 50/26, G16H 20/30, G16H 20/60

(54) **METHOD AND DEVICE FOR SHARING PERSONAL HEALTH RECORD**

(30) Priority: 10.10.2023 KR 20230134663
(71) Applicant: Seegene, Inc., Seoul 05548 (KR)
(72) Inventor: KANG, Seunggoo, Seongnam-si, Gyeonggi-do 13555 (KR); CHO, Nakhoon, Seoul 06004 (KR); KIM, Jaeil, Seoul 04774 (KR); CHO, Young O, Seoul 04998 (KR)
(74) Representative: Viering, Jentschura & Partner mbB Patent- und Rechtsanwälte
(86) International application number: PCT/KR2024/015319
(87) International publication number: WO 2025/079975

(57) **Abstract**

A method and apparatus for sharing a personal health record are provided. A method for sharing a personal health record according to one embodiment includes: acquiring personal health records from user devices; performing a shared user authentication process for users who provided the personal health records to share the personal health records; and permitting sharing of the personal health records among users who have obtained an authentication level, wherein the performing of the shared user authentication process may include comparing a medical record of a user acquired from a healthcare provider recorded in the personal health record with the personal health record acquired from the user device, and assigning an authentication level to the user according to a degree of matching resulting from the comparing.

## Description

### TECHNICAL FIELD

The present disclosure relates to a method for sharing a personal health record and a personal health record sharing device capable of sharing a personal health record.

### BACKGROUND OF THE INVENTION

A personal health record (PHR) is medical and health data that is proactively managed by an individual, and broadly refers to a system for managing medical and health records. Recently, personal health records are managed in association with electronic medical records (EMRs) and are integrated with electronic medical records of healthcare providers.

Personal health records are information closely related to medical services and medical research, and may be used for efficient health management and treatment purposes of users or for research purposes. As such, personal health records are increasingly utilized for the purposes of users' health management, treatment, and various research purposes.

However, since personal health records include sensitive personal information, care must be taken to prevent personal health records from being indiscriminately disclosed or leaked without the consent of the user. In particular, personal health records of other users must not be indiscriminately redistributed or modified. In addition, personal health records must not be used for research purposes without the consent of the user, and even when used for research purposes, they must be utilized within a limited scope so as not to cause harm to the user who is the subject of the personal health record.

In particular, modification of a personal health record without the consent of the user who is the subject of the personal health record may represent the health condition of the user differently from the actual health condition, thereby causing harm to the user who is the subject of the personal health record due to inaccurate information. In addition, when health management is performed using inaccurate personal health records, it is not possible to properly analyze the health condition of the user, and the accuracy of health management and treatment of the user is reduced. Furthermore, when a personal health record is arbitrarily modified, objectivity is reduced due to information modified differently from reality, and thus the personal health record cannot be utilized for research purposes.

In order to achieve such purposes of utilizing personal health records, information included in the personal health records must have reliability. Meanwhile, although information included in a personal health record may be input based on objective information, there are also items that are directly input by the user, and thus not all information can be objective.

Therefore, reliability of information input into personal health records is required, and allowing personal health records input by users to be viewed by other users, redistributed by other users, or modified by other users should be permitted only in exceptional cases.

### DETAILED DESCRIPTION OF THE DISCLOSURE

### TECHNICAL PROBLEMS

A problem to be solved in the present specification is to acquire a personal health record from a user and to share the acquired personal health record with other users.

Another problem to be solved in the present specification is to acquire a personal health record having high reliability from a user.

Still another problem to be solved in the present specification is to allow a user whose reliability has been secured to utilize personal health records of other users.

However, the problems to be solved described in the present specification are not limited to the problems mentioned above, and other problems not mentioned may be clearly understood by a person having ordinary skill in the art to which the present disclosure pertains from the following description.

### MEANS FOR SOLVING PROBLEMS

A method for sharing a personal health record according to one embodiment is a method for sharing a personal health record acquired from a user device by a personal health record sharing device using a memory, a processor, and one or more programs stored in the memory and configured to be executed by the processor, the method comprising: acquiring, by the processor, personal health record from user device; performing, by the processor, a shared user authentication process for user who provided the personal health record to share the personal health record; and permitting, by the processor, sharing of the personal health records among users who have obtained an authentication level in performing the shared user authentication process, and the performing of the shared user authentication process comprises: comparing, by the processor, a medical record of the user acquired from a healthcare provider and recorded in the personal health record with the personal health record acquired from the user device; and assigning, by the processor, an authentication level to the user according to a degree of matching resulting from the comparing.

In one embodiment, the personal health record comprises at least one selected from the group consisting of: symptom information, disease information, onset timing, clinical information on the disease, post-treatment information following the clinical care, treatment information for the disease, post-treatment information following the treatment, prescription information for treatment, medication intake information of the user, medication purchase information of the user, medication intake review information based on the medication purchase record, and daily health record information of the user, and wherein the personal health record is input by the user.

In one embodiment, the medical record comprises at least one selected from the group consisting of: symptom information, disease information, onset timing, clinical information on the disease, treatment information for the disease, post-treatment information following the treatment, prescription information for treatment, medication administration information for the user, medical department information, patient name, medical professional in charge, and recommended management methods for managing the disease of the user, and wherein the medical record is input by the healthcare provider.

In one embodiment, the assigning of the authentication level to the user comprises providing the user, according to the authentication level, with at least one authority selected from the group consisting of a viewing authority for personal health records among the users, a redistribution authority for other user's personal health records, and a derivative modification authority for other user's personal health records.

In one embodiment, the viewing authority is granted when the authentication level for the user is equal to or higher than a viewing authentication level threshold which is preset.

In one embodiment, the redistribution authority is granted when the authentication level for the user is equal to or higher than a redistribution authentication level threshold which is preset.

In one embodiment, the derivative modification authority is granted when the authentication level for the user is equal to or higher than a derivative modification authentication level threshold which is preset.

In one embodiment, the assigning of the authentication level to the user comprises: comparing a recommended treatment method for treating the user's disease included in the medical record with treatment process information for treating the user's disease included in the personal health record; and assigning a higher authentication level as the recommended treatment method and the treatment process information match more with each other.

In one embodiment, the assigning of the authentication level to the user comprises: comparing medication administration information for the user included in the medical record with medication intake information of the user included in the personal health record; and assigning a higher authentication level as the medication administration information and the medication intake information match more with each other.

In one embodiment, the assigning of the authentication level to the user comprises: comparing first sequence data including time-series information among information included in the medical record with second sequence data including time-series information among information included in the personal health record; and assigning a higher authentication level as the first sequence data and the second sequence data match more with each other.

In one embodiment, the assigning of the authentication level to the user comprises: comparing recommended exercise and diet information for treating the user's disease included in the personal health record; and assigning a higher authentication level as the recommended exercise and diet information and the exercise and the diet records match more with each other.

In one embodiment, the method further comprising determining whether disease information included in the personal health record corresponds to pre-designated disease information, and the performing of the shared user authentication process is performed only when the disease information corresponds to the pre-designated disease information.

In one embodiment, the permitting of sharing of the personal health record among users comprises providing a function to transmit and receive privacy messages between the users allowed to share the personal health record.

In one embodiment, the privacy message may be a service capable of transmitting and receiving data and messages related to the personal health record.

A personal health record sharing device according to another embodiment comprising: a processor; a memory; and a computer program loaded in the memory and executed by the processor, wherein the computer program comprises instructions for: acquiring personal health record from user device; performing a shared user authentication process for a user who provided the personal health records to share the personal health record; and permitting sharing of the personal health records among users who have obtained an authentication level, and the instructions for performing the shared user authentication process comprise instructions for: comparing a medical record of the user acquired from a healthcare provider recorded in the personal health record with the personal health record acquired from the user device; and assigning an authentication level to the user according to a degree of matching resulting from the comparing.

### EFFECTS OF INVENTION

According to one embodiment of the present disclosure, by acquiring personal health records from users, it is possible to provide a service that allows users to share personal health records with each other.

According to one embodiment of the present disclosure, by assigning authentication levels to users who have provided personal health records having high reliability, it is possible to induce input of personal health records having high reliability.

According to one embodiment of the present disclosure, it is possible to promote utilization of personal health records in various manners.

### BRIEF DESCRIPTION OF THE DRAWINGS

FIG. 1 is a flowchart of a method for sharing a personal health record according to one embodiment.
FIG. 2 is a diagram schematically illustrating a network relationship between a personal health record sharing device and a user device.
FIG. 3 is a flowchart illustrating detailed steps of a shared user authentication process.
FIG. 4 is a diagram for explaining an operation of determining an authentication level by using a personal health record acquired from a user and a medical record acquired from a healthcare provider.
FIG. 5 is a diagram for explaining authorities according to authentication levels of users permitted to share personal health records.
FIG. 6 is a hardware configuration diagram of a personal health record sharing device.

### BEST MODE FOR IMPLEMENTING THE INVENTION

Advantages and features of the present disclosure, and methods for achieving the same, will become apparent with reference to embodiments described in detail below in conjunction with the accompanying drawings. However, the present disclosure is not limited to the embodiments disclosed below and may be implemented in various different forms. The embodiments are provided only to complete the disclosure of the present invention and to fully inform a person having ordinary skill in the art to which the present invention pertains of the scope of the invention, and the present invention is defined only by the scope of the claims.

In describing the embodiments of the present disclosure, detailed descriptions of well-known functions or configurations will be omitted when it is determined that such descriptions unnecessarily obscure the gist of the present invention. In addition, terms described below are defined in consideration of functions in the embodiments of the present invention, and may vary depending on intentions or practices of users or operators. Therefore, the definitions should be determined based on the contents throughout this specification.

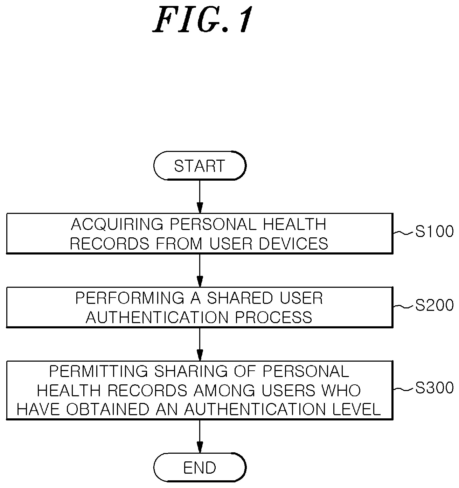

FIG. 1 is a flowchart of a method for sharing a personal health record according to one embodiment.

Referring to FIG. 1, the method for sharing a personal health record according to one embodiment may include acquiring a personal health record from a user device in step S100, performing a shared user authentication process in step S200, and sharing the personal health record among users who have obtained an authentication level in step S300. The method for sharing a personal health record according to one embodiment may be performed by a personal health record sharing device, and in this case, the personal health record sharing device may be a computing device.

Specifically, in step S100, the personal health record sharing device may acquire a personal health record of a user and store the acquired personal health record. The personal health record is information related to health of the user input by the user, and may be information input through the user device. In one embodiment, the personal health record may include at least one selected from the group consisting of: symptom information of the user, disease information, onset timing, medical examination/treatment information on the disease, post-treatment information following the medical examination/treatment, treatment information for treating the disease, post-treatment information following the treatment, prescription information for treatment, medication intake information of the user, medication purchase information of the user, medication intake review information based on a medication purchase record of the user, and daily health record information of the user.

In one embodiment, the personal health record may include a molecular diagnostic test result. In this case, the molecular diagnostic test result may be a diagnostic test result obtained by a nucleic acid amplification reaction or polymerase chain reaction (PCR).

When the personal health record sharing device acquires the personal health record from the user device, the personal health record sharing device may parse contents included in the personal health record and classify and store the parsed information based on the parsed information. For example, the personal health record sharing device may classify whether contents included in the personal health record correspond to symptom information of the user, disease information, onset timing, medical examination/treatment information on the disease, post-treatment information following the medical examination/treatment, treatment information for treating the disease, post-treatment information following the treatment, prescription information for treatment, medication intake information of the user, medication purchase information of the user, medication intake review information based on a medication purchase record of the user, or daily health record information of the user, and store the classified information in a database (DB).

Information related to treatment (medical examination) and/or medications according to prescriptions may be provided not only from the user device, but also from a healthcare institution device or a public healthcare institution device that manages medical records. The healthcare institution device or the public healthcare institution device may provide medical records for which user consent has been completed to the personal health record sharing device.

In step S200, a shared user authentication process may be performed. The personal health record sharing device may perform the shared user authentication process for sharing personal health records of users based on the acquired personal health records. In this case, the personal health record sharing device may assign an authentication level to each user based on the shared user authentication process. The personal health record sharing device may assign different authentication levels to different users.

In step S300, sharing of the personal health record among users who have obtained authentication levels may be permitted. Here, permitting sharing means allowing a user to view, redistribute, or perform derivative modification of personal health records of other users. That is, permitting sharing means permitting users who have obtained authentication levels to utilize personal health records that have already been acquired.

In one embodiment, permitting sharing may include providing a function for transmitting and receiving privacy messages (direct messages) between users permitted to share personal health records. In this case, the privacy message (direct message) may be a service capable of transmitting and receiving data related to the personal health record and messages related to the personal health record.

The personal health record sharing device may provide at least one authority selected from viewing authority, redistribution authority, and derivative modification authority for personal health records among users who have obtained authentication levels. In this case, the personal health record sharing device may provide different authorities according to authentication levels. Specifically, when permitting sharing of personal health records, the personal health record sharing device may assign different authentication levels based on personal health records provided by users. In addition, the personal health record sharing device may provide authorities according to authentication levels to users. The personal health record sharing device may assign an authentication level that allows broader authorities to a user who has provided a personal health record having high reliability.

According to one embodiment, since the personal health record sharing device provides different authorities to users according to authentication levels, users may provide personal health records having higher reliability in order to obtain broader authorities.

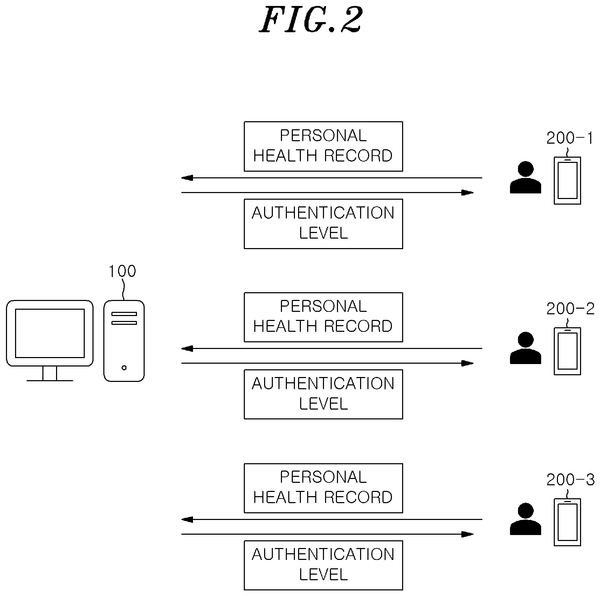

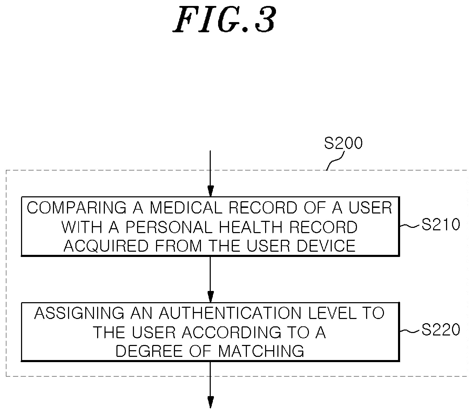

FIG. 2 is a diagram schematically illustrating a network relationship between a personal health record sharing device and a user device, and FIG. 3 is a flowchart illustrating detailed steps of a shared user authentication process.

Referring to FIG. 2, a personal health record sharing device (100) may acquire a personal health record (PHR) from user devices (200-1 to 200-3), and may assign an authentication level for each user based on the acquired personal health record.

Specifically, when the personal health record sharing device (100) acquires a personal health record from the user devices (200-1 to 200-3), the personal health record sharing device (100) may assign an authentication level to a user based on a medical record of the user who provided the personal health record.

The medical record may be provided from a healthcare institution device or a public healthcare institution device that manages medical records. The healthcare institution device or the public healthcare institution device may provide the medical record for which user consent has been completed to the personal health record sharing device (100).

Herein, the medical record may include at least one selected from the group consisting of: symptom information of the user input by a healthcare provider, disease information, onset timing, medical examination/treatment information on the disease, treatment information for treating the disease, post-treatment information following the treatment, prescription information for treatment, medication administration information for the user, medical department information, patient name, medical professional in charge, and recommended management methods for managing the disease of the user. In one embodiment, the medical record may include a molecular diagnostic test result. In this case, the molecular diagnostic test result may be a diagnostic test result obtained by a nucleic acid amplification reaction or polymerase chain reaction (PCR).

In one embodiment, the medical record may be an electronic medical record (EMR), but is not limited thereto.

In one embodiment, the personal health record sharing device (100) may acquire the medical record of the user from a healthcare provider identified in the personal health record.

When the personal health record sharing device (100) acquires the personal health record, the personal health record sharing device (100) may request the medical record of the user from the healthcare provider recorded in the personal health record, and may acquire the medical record from the healthcare provider.

In this case, the personal health record sharing device (100) may acquire the medical record after presenting information indicating that consent of the user has been obtained to the healthcare provider.

In another embodiment, regardless of whether the healthcare provider is recorded in the personal health record, the personal health record sharing device (100) may acquire, periodically or aperiodically, a medical record of a user for whom consent has been completed from a healthcare provider.

The personal health record sharing device (100) may compare the personal health record of the user who acquired the personal health record with the medical record, and may analyze to what extent contents described in the personal health record match the medical record. The personal health record sharing device (100) may assign an authentication level to the user based on a degree of matching. The personal health record sharing device (100) may determine the authentication level of the user according to preset criteria.

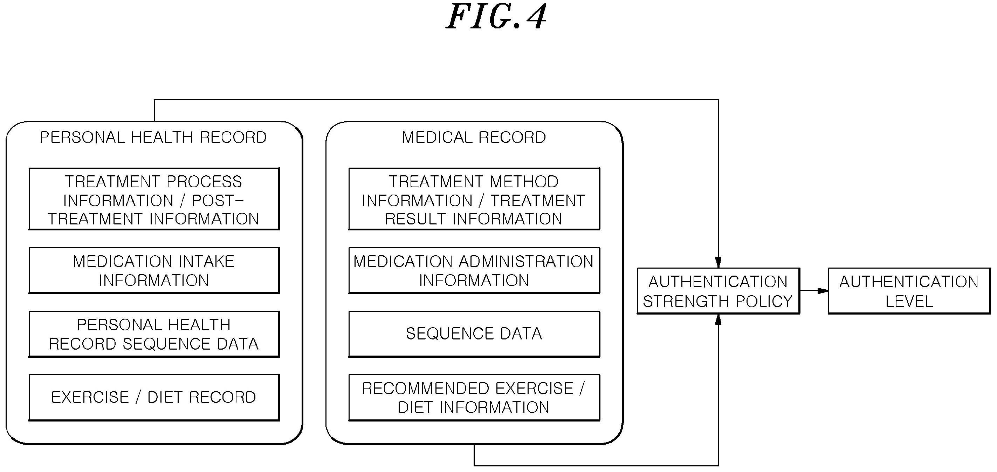

A detailed operation of assigning an authentication level to a user will be described with reference to FIGS. 3 and 4.

First, in step S210 of FIG. 3, the medical record of the user acquired from a healthcare provider identified in the personal health record and the personal health record acquired from the user device may be compared. That is, the personal health record sharing device may compare information input by the user with information input by the healthcare provider.

For example, the personal health record sharing device may compare at least one of: symptom information of the user, disease information, onset timing, medical examination/treatment information on the disease, post-treatment information following the medical examination/treatment, treatment information for treating the disease, post-treatment information following the treatment, prescription information for treatment, medication intake information of the user, medication purchase information of the user, medication intake review information based on a medication purchase record of the user, and daily health record information of the user, which are included in the personal health record and input by the user, with at least one of: symptom information of the user, disease information, onset timing, medical examination/treatment information on the disease, treatment information for treating the disease, post-treatment information following the treatment, prescription information for treatment, medication administration information for the user, medical department information, patient name, medical professional in charge, and recommended management methods for managing the disease of the user, which are included in the medical record.

When performing the comparing operation, the personal health record sharing device may compare information corresponding to the same category with each other. The personal health record sharing device may compare each item of the medical record with each item of the personal health record. The personal health record sharing device may determine whether compared information matches by comparing items corresponding to the same category.

For example, the personal health record sharing device may compare disease information included in the medical record with disease information included in the personal health record. Alternatively, the personal health record sharing device may compare onset timing included in the personal health record with onset timing included in the medical record, or may compare medical examination/treatment information on the disease included in the personal health record with medical examination/treatment information on the disease of the user included in the medical record. As described above, the personal health record sharing device may compare corresponding information with each other using criteria in which a comparison target is pre-defined.

In step S220, an authentication level may be assigned to the user according to a degree of matching resulting from the comparing. The personal health record sharing device may assign an authentication level to the user according to the degree of matching of the compared information.

In one embodiment, since the personal health record is input by the user, an error may occur or reliability may be low, but when the personal health record is compared with the medical record, it may be determined whether contents described in the personal health record have been accurately input.

Since the medical record is information input by the healthcare provider, the medical record may be determined to have relatively higher credibility than the personal health record. Accordingly, when information described in the personal health record matches information included in the medical record, since it is similar to the medical record having high credibility, credibility of the information may be determined to be high.

In addition, the personal health record may be information by which it is possible to determine whether the user has performed appropriate treatment and health management. That is, the personal health record sharing device may determine how appropriately the user performed prescriptions or recommended methods of the healthcare provider by comparing information included in the personal health record, such as medical examination/treatment information on the disease input by the user, post-treatment information following the medical examination/treatment, treatment information for treating the disease, post-treatment information following the treatment, prescription information for treatment, medication intake information of the user, medication purchase information of the user, medication intake review information based on the medication purchase record of the user, and daily health record information of the user, with information included in the medical record.

The personal health record sharing device may assign an authentication level that provides broader authorities to a user who prepared a personal health record having high credibility according to a degree of matching resulting from the comparing, or to a user who well performed instructions received from a healthcare provider.

In another embodiment, when determining an authentication level, the shared user authentication process may additionally use external reputation information indicating information on a reputation of the user, recommender reference information, and degree information.

FIG. 4 is a diagram for explaining an operation of determining an authentication level by using a personal health record acquired from a user and a medical record acquired from a healthcare provider.

As shown in FIG. 4, the personal health record sharing device may determine an authentication level by comparing the personal health record with the medical record based on an authentication threshold policy.

According to the authentication level, an authority may be a viewing authority for personal health records among users, a redistribution authority for another user's personal health record, or a derivative modification authority for another user's personal health record.

A user who has been assigned an authentication level for a viewing authority may view personal health records provided by other users.

A user who has been assigned an authentication level for a redistribution authority may redistribute personal health records provided by other users. In this case, the user who has been assigned the redistribution authority may also view personal health records provided by other users.

A user who has been assigned an authentication level for a derivative modification authority may perform derivative modification of personal health records provided by other users. In this case, the user who has been assigned the derivative modification authority may also redistribute or view personal health records provided by other users. That is, the derivative modification authority may include the redistribution authority and the viewing authority, and the redistribution authority may include the viewing authority.

The authentication level may be determined according to the authentication threshold policy.

Here, the authentication threshold policy is criteria for determining an authentication level according to a degree of matching of compared information. That is, the authentication threshold policy may be classified into a viewing authentication level threshold, a redistribution authentication level threshold, and a derivative modification authentication level threshold.

The viewing authority may be granted when the authentication level for the user is equal to or higher than the viewing authentication level threshold among preset authentication threshold policies. The redistribution authority may be granted when the authentication level for the user is equal to or higher than the redistribution authentication level threshold among the preset authentication threshold policies. The derivative modification authority may be granted when the authentication level for the user is equal to or higher than the derivative modification authentication level threshold among the preset authentication threshold policies.

The personal health record sharing device may calculate a degree of matching by comparing information included in the personal health record with information included in the medical record, and may determine which authentication level threshold among the viewing authentication level threshold, the redistribution authentication level threshold, and the derivative modification authentication level threshold the degree of matching is higher than.

For example, the personal health record sharing device may assign a higher authentication level to the user as information on a pharmaceutical product/medication included in the personal health record and information on a pharmaceutical product/medication included in the medical record become identical. Alternatively, the personal health record sharing device may assign a higher authentication level to the user as a treatment course included in the personal health record and treatment method information included in the medical record become identical. Alternatively, the personal health record sharing device may assign a higher authentication level to the user as an exercise/diet record included in the personal health record and recommended exercise/diet included in the medical record become identical. Such examples will be described in detail.

Specifically, the personal health record sharing device may compare treatment information included in the personal health record with treatment information included in the medical record. The treatment information included in the personal health record may mean information related to a treatment process input by the user. The treatment information included in the medical record may be information related to a treatment method among information input by a medical professional, a medical assistant, or an office worker of a healthcare provider. The personal health record sharing device may assign a higher authentication level as the treatment information included in the personal health record and the treatment information included in the medical record become identical.

The personal health record sharing device may compare post-treatment information included in the personal health record with post-treatment information included in the medical record. The post-treatment information may include a treatment photo of the user, text, a review, a link address connected to treatment review information, or social network service (SNS) information related to treatment review. The post-treatment information may include a medical examination/treatment record on treatment results of the user input for the user by the healthcare provider, a medical certificate, or an opinion letter. The personal health record sharing device may assign a higher authentication level as the post-treatment information included in the personal health record and the post-treatment information included in the medical record become identical.

The personal health record sharing device may compare medication intake information included in the personal health record with medication administration information included in the medical record. The medication intake information may be intake information of a pharmaceutical product/medication input by the user. The medication administration information included in the medical record may be information on a pharmaceutical product/medication prescribed by the healthcare provider, or information on a pharmaceutical product/medication sold to the user at a pharmacy. The personal health record sharing device may assign a higher authentication level to the user as information on the pharmaceutical product/medication included in the personal health record and information on the pharmaceutical product/medication included in the medical record become identical.

The personal health record sharing device may compare personal health record sequence data included in the personal health record with sequence data included in the medical record. The personal health record sequence data may include time-series information related to the personal health record. For example, the personal health record sequence data may include symptom onset information input in temporal order, medication administration information input in temporal order, and treatment review input in temporal order. The sequence data including time-series information may include, from onset of a disease to treatment, or deterioration/improvement of personal health. In the health or medical field, sequence data may be used as important data for analyzing a health condition of the user. The personal health record sharing device may assign a higher authentication level to the user as the sequence data included in the personal health record and the sequence data included in the medical record become identical. In this case, the personal health record sharing device may assign a higher authentication level as a temporal period of the sequence data becomes longer.

The personal health record sharing device may compare an exercise/diet record (daily health record information) included in the personal health record with recommended exercise/diet information (daily health record information) included in the medical record. The exercise/diet record (daily health record information) may include food intake, snack intake, and intake record of health supplements. The personal health record sharing device may assign a higher authentication level to the user as the exercise/diet record (daily health record information) included in the personal health record and the recommended exercise/diet (recommended management methods) included in the medical record become identical.

Alternatively, when a degree of matching by comparing information included in the personal health record with information included in the medical record is higher than the viewing authentication level threshold but lower than the redistribution authentication level threshold and the derivative modification authentication level threshold, the personal health record sharing device may determine an authentication level as the viewing authority.

Alternatively, when a degree of matching by comparing information included in the personal health record with information included in the medical record is higher than the viewing authentication level threshold and the redistribution authentication level threshold but lower than the derivative modification authentication level threshold, the personal health record sharing device may determine an authentication level as the redistribution authority.

Alternatively, when a degree of matching by comparing information included in the personal health record with information included in the medical record is higher than the viewing authentication level threshold, the redistribution authentication level threshold, and the derivative modification authentication level threshold, the personal health record sharing device may determine an authentication level as the derivative modification authority.

Meanwhile, in another embodiment, the personal health record sharing device may assign an authentication level for each personal health record. When an authentication level is assigned to a personal health record, it means that credibility of the corresponding personal health record is high. A personal health record to which an authentication level is assigned may be permitted to be viewed, permitted to be redistributed, or permitted to be subjected to derivative modification. In this case, a personal health record that has obtained an authentication level to which a viewing authority is granted may be viewable by other users. Alternatively, a personal health record that has obtained an authentication level to which a redistribution authority is granted may be viewable and redistributable to other users. Alternatively, a personal health record that has obtained an authentication level to which a derivative modification authority is granted may be viewable, redistributable, and derivatively modifiable to other users.

The personal health record sharing device may not assign any authentication level when a degree of matching by comparing information included in the personal health record with information included in the medical record is not higher than at least one of the viewing authentication level threshold, the redistribution authentication level threshold, and the derivative modification authentication level threshold.

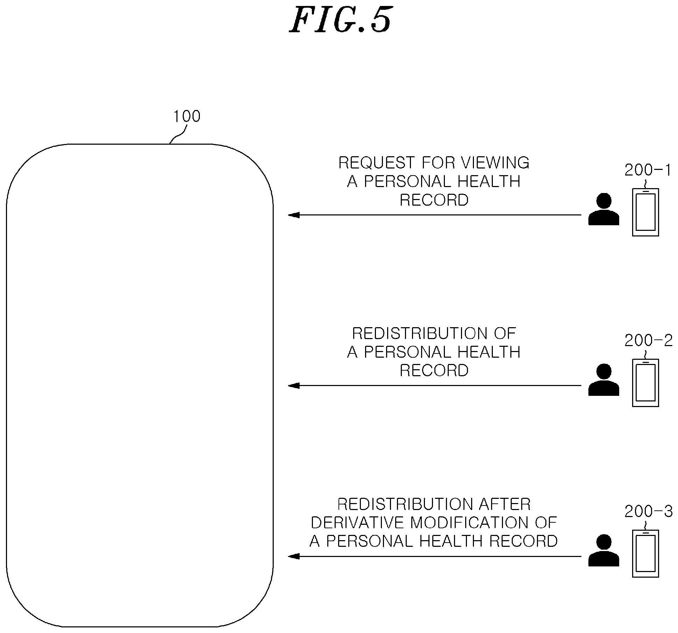

FIG. 5 is a diagram for explaining authorities according to authentication levels of users permitted to share personal health records.

Referring to FIG. 5, the personal health record sharing device may receive, from a user to whom an authentication level has been assigned, a request to view a personal health record of another user, may receive redistribution of a personal health record, or may acquire a derivatively modified personal health record.

For example, when a user is assigned an authentication level for a viewing authority, the personal health record sharing device may receive a request for viewing a personal health record from a user device (200-1), and may provide the requested personal health record. The device may collect and store personal health records acquired from users, and may provide users with a list of summarized information classified by disease type of users, or by physical information, health information, or the like of users.

Users may request desired personal health records from among the provided summarized information list. Alternatively, users may request desired disease information, desired user age, desired user gender, or desired sequence data, and the personal health record sharing device may provide personal health records including information identical or similar to the requested information based on the request.

In addition, when a user is assigned an authentication level for a redistribution authority, the user may view and redistribute the acquired personal health record. A user device (200-2) may request viewing of a personal health record of another user and acquire the personal health record, and may redistribute the acquired personal health record. In this case, redistribution of the acquired personal health record may mean distributing the personal health record on a platform operated by the personal health record sharing device or distributing the personal health record to an external server.

Further, redistribution may mean distributing the personal health record without modifying contents of the personal health record. In one embodiment, redistribution may include an information hiding operation of covering some contents without changing contents of the personal health record, such as blurring (blur) or mosaicking. For example, redistribution may include redistributing a personal health record in which personal identification information of a user included in the personal health record is mosaicked.

According to the present embodiment, since a user who has been granted redistribution authority for a personal health record may freely distribute personal health records of other users, the user may utilize personal health records of other users by citing or using them as reference information in papers or the like.

In addition, when a user is assigned an authentication level for a derivative modification authority, the user may modify a portion of an acquired personal health record of another user and then redistribute the modified personal health record. The user device (200-3) may request viewing of a personal health record of another user and acquire the personal health record, and may perform derivative modification of the acquired personal health record.

Here, the derivative modification authority refers to a personal health record in which some information of an original personal health record is modified. That is, the derivative modification authority is an authority to distribute a modified personal health record derived from an original personal health record. The derivative modification authority may include a reproduction authority and an authority to produce a derivative work.

However, not all information included in the personal health record may be modified, and only pre-designated items may be modified. In addition, even when modifying pre-designated items, such modification may not exceed a pre-defined numerical range.

As described above, a personal health record partially modified by the derivative modification authority may be redistributed to a platform managed by the personal health record sharing device.

The personal health record sharing device may provide the derivative modification authority to a user having high credibility because the authentication level of the user is determined to be high through the shared user authentication process.

A personal health record may include various types of information, such as profile information of a user, medical information, health information, information generated during a treatment process, and clinical information. Therefore, when such information is indiscriminately distributed, it may cause harm to a user who is a subject of the personal health record. In particular, when a personal health record is arbitrarily modified, reliability of the personal health record is degraded and incorrect information may be provided, and thus modification of the personal health record requires more careful attention.

Accordingly, authority for redistributing a personal health record of another user or modifying a personal health record of another user should be granted only to limited users.

The method for sharing a personal health record according to the present embodiment compares and matches a personal health record provided by a user with a medical record acquired from a healthcare provider to determine whether contents included in the personal health record of the user are reliable information, and through this, it is possible to determine that the corresponding user is capable of providing information having high credibility. According to one embodiment, by providing stepwise authorities to a user having high credibility, it is possible to receive personal health records having higher credibility from users in order to obtain broader authorities.

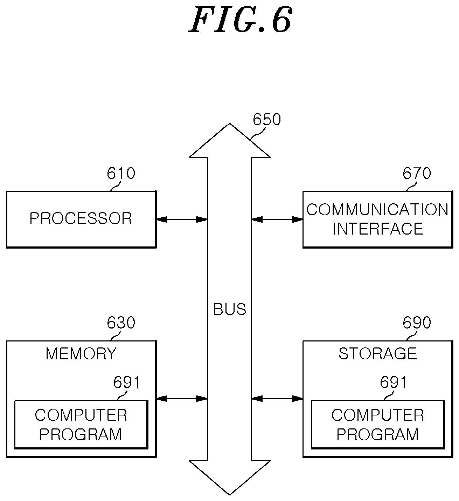

Hereinafter, an exemplary computing device (600) capable of implementing a community service management device described in various embodiments of the present invention will be described with reference to FIG. 6. In the present embodiment, the computing device (600) may be the community service management device (100) of FIG. 1.

FIG. 6 is an exemplary hardware configuration diagram illustrating the computing device (600).

As illustrated in FIG. 6, a computing device (600) may include one or more processors (610), a bus (650), a communication interface (670), a memory (630) for loading a computer program (691) executed by the processor (610), and a storage (690) for storing the computer program (691). However, only components related to embodiments of the present invention are illustrated in FIG. 6. Accordingly, a person having ordinary skill in the art to which the present invention pertains may understand that other general-purpose components may be further included in addition to the components illustrated in FIG. 6.

The processor (610) controls overall operations of each component of the computing device (600). The processor (610) may include at least one of a CPU (Central Processing Unit), an MPU (Micro Processor Unit), an MCU (Micro Controller Unit), a GPU (Graphic Processing Unit), or any type of processor well known in the technical field of the present invention. In addition, the processor (610) may perform operations for at least one application or program for executing methods/operations according to various embodiments of the present invention. The computing device (600) may include one or more processors.

The memory (630) stores various data, instructions, and/or information. The memory (630) may load one or more programs (691) from the storage (690) in order to execute methods/operations according to various embodiments of the present invention. For example, when the computer program (691) is loaded into the memory (630), logic (or modules) such as those illustrated in FIG. 4 may be implemented on the memory (630). An example of the memory (630) may be RAM, but is not limited thereto.

The bus (650) provides a communication function between components of the computing device (600). The bus (650) may be implemented as various types of buses, such as an address bus, a data bus, and a control bus.

The communication interface (670) supports wired and wireless Internet communication of the computing device (600). The communication interface (670) may also support various communication methods other than Internet communication. To this end, the communication interface (670) may include communication modules well known in the technical field of the present invention.

The storage (690) may non-transitorily store one or more computer programs (691). The storage (690) may include a non-volatile memory such as ROM (Read Only Memory), EPROM (Erasable Programmable ROM), EEPROM (Electrically Erasable Programmable ROM), flash memory, a hard disk, a removable disk, or any type of computer-readable recording medium well known in the technical field to which the present invention pertains.

The computer program (691) may include one or more instructions in which methods/operations according to various embodiments of the present invention are implemented. When the computer program (691) is loaded into the memory (630), the processor (610) may perform methods/operations according to various embodiments of the present invention by executing the one or more instructions.

In one embodiment, the computer program may include: instructions for acquiring personal health records from user devices; instructions for performing a shared user authentication process for users who provided the personal health records to share the personal health records; and instructions for permitting sharing of the personal health records among users who have obtained an authentication level, and the instructions for performing the shared user authentication process may include: instructions for comparing a medical record of a user acquired from a healthcare provider recorded in the personal health record with the personal health record acquired from the user device; and instructions for assigning an authentication level to the user according to a degree of matching resulting from the comparing.

Meanwhile, the methods according to the various embodiments described above may be implemented in the form of a computer program stored in a computer-readable recording medium programmed to perform each step of the methods, and may also be implemented in the form of a computer-readable recording medium storing a computer program programmed to perform each step of the methods.

The above description is merely illustrative of the technical idea of the present disclosure, and those skilled in the art to which the present disclosure pertains will be able to make various modifications and variations without departing from the essential quality of the present disclosure. Accordingly, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

## Claims

1. A method for sharing a personal health record (PHR) acquired from a user device by a personal health record sharing device using a memory, a processor, and one or more programs stored in the memory and configured to be executed by the processor, the method comprising:
acquiring, by the processor, personal health record from the user device;
performing, by the processor, a shared user authentication process for a user who provided the personal health record to share the personal health record; and
permitting, by the processor, sharing of the personal health records among users who have obtained an authentication level in the performing of the shared user authentication process,
wherein the performing of the shared user authentication process comprises:
comparing, by the processor, a medical record of the user acquired from a healthcare provider and recorded in the personal health record with the personal health record acquired from the user device; and
assigning, by the processor, an authentication level to the user according to a degree of matching resulting from the comparing.

2. The method of claim 1, wherein the personal health record comprises at least one selected from the group consisting of: symptom information, disease information, onset timing, clinical information on the disease, post-treatment information following the clinical care, treatment information for the disease, post-treatment information following the treatment, prescription information for treatment, medication intake information of the user, medication purchase information of the user, medication intake review information based on the medication purchase record, and daily health record information of the user, and wherein the personal health record is input by the user.

3. The method of claim 1, wherein the medical record comprises at least one selected from the group consisting of: symptom information, disease information, onset timing, clinical information on the disease, treatment information for the disease, post-treatment information following the treatment, prescription information for treatment, medication administration information for the user, medical department information, patient name, medical professional in charge, and recommended management methods for managing the disease of the user, and wherein the medical record is input by the healthcare provider.

4. The method of claim 1, wherein the assigning of the authentication level to the user comprises providing the user, according to the authentication level, with at least one authority selected from the group consisting of a viewing authority for personal health records among the users, a redistribution authority for other user's personal health records, and a derivative modification authority for other user's personal health records.

5. The method of claim 4, wherein the viewing authority is granted when the authentication level for the user is equal to or higher than a viewing authentication level threshold which is preset.

6. The method of claim 4, wherein the redistribution authority is granted when the authentication level for the user is equal to or higher than a redistribution authentication level threshold which is preset.

7. The method of claim 4, wherein the derivative modification authority is granted when the authentication level for the user is equal to or higher than a derivative modification authentication level threshold which is preset.

8. The method of claim 1, wherein the assigning of the authentication level to the user comprises:
comparing a recommended treatment method for treating the user's disease included in the medical record with treatment process information for treating the user's disease included in the personal health record; and
assigning a higher authentication level as the recommended treatment method and the treatment process information match more with each other.

9. The method of claim 1, wherein the assigning of the authentication level to the user comprises:
comparing medication administration information for the user included in the medical record with medication intake information of the user included in the personal health record; and
assigning a higher authentication level as the medication administration information and the medication intake information match more with each other.

10. The method of claim 1, wherein the assigning of the authentication level to the user comprises:
comparing first sequence data including time-series information among information included in the medical record with second sequence data including time-series information among information included in the personal health record; and
assigning a higher authentication level as the first sequence data and the second sequence data match more with each other.

11. The method of claim 10, wherein the assigning of the higher authentication level comprises assigning a higher authentication level as a time period of the time-series informations becomes longer.

12. The method of claim 1, wherein the assigning of the authentication level to the user comprises:
comparing recommended exercise and diet information for treating the user's disease included in the medical record with exercise records and diet records of the user included in the personal health record; and
assigning a higher authentication level as the recommended exercise and diet information and the exercise and diet records match more with each other.

13. The method of claim 1, further comprising determining whether disease information included in the personal health record corresponds to pre-designated disease information,
wherein the performing of the shared user authentication process is performed only when the disease information corresponds to the pre-designated disease information.

14. The method of claim 1, wherein the permitting of the sharing of the personal health record among users comprises providing a function to transmit and receive privacy messages between the users allowed to share the personal health record.

15. The method of claim 14, wherein the privacy message is a service capable of transmitting and receiving data and messages related to the personal health record.

16. A personal health record sharing device comprising:
a processor;
a memory; and
a computer program stored in the memory and executed by the processor,
wherein the computer program comprises instructions for:
acquiring personal health record from user device;
performing a shared user authentication process for a user who provided the personal health record to share the personal health record; and
permitting sharing of the personal health records among users who have obtained an authentication level,
wherein the instructions for performing the shared user authentication process comprise instructions for:
comparing a medical record of a user acquired from a healthcare provider recorded in the personal health record with the personal health record acquired from the user device; and
assigning an authentication level to the user according to a degree of matching resulting from the comparing.

17. A computer-readable recording medium storing a program for implementing the personal health record sharing method according to any on of claim 1 to 15.

18. A program stored in a computer-readable recording medium for implementing the personal health record sharing method according to any on of claim 1 to 15.
